# EUROPEAN PATENT SPECIFICATION

(11) **EP 2 046 294 B1**
(45) Date of publication and mention of the grant of the patent: **21.12.2011**
(21) Application number: 07805082.0
(22) Date of filing: 09.07.2007
(51) Int. Cl.: A61K 9/14, A61K 9/16

(54) **THERMOCHROMIC COMPOSITIONS FOR SKIN APPLICATION**
THERMOCHROME ZUSAMMENSETZUNGEN ZUR ANWENDUNG AUF DER HAUT
COMPOSITIONS THERMOCHROMIQUES Á APPLIQUER SUR LA PEAU

(30) Priority: 27.07.2006 US 494252
(43) Date of publication of application: 15.04.2009
(73) Proprietor: KIMBERLY-CLARK WORLDWIDE, INC., Neenah, WI 54956 (US)
(72) Inventor: MACDONALD, John Gavin, Decatur, Georgia 30033 (US); SCHORR, Phillip, Atlanta, Georgia 30350 (US); AREHART, Kelly D., Roswell, Georgia 30075 (US); BROWN, Tameka S., Lilburn, Georgia 30047 (US); LINTON, Lisa A., Alpharetta, Georgia 30022 (US); OSLUND, Susan G., Roswell, Georgia 30075 (US)
(74) Representative: Beacham, Annabel Rose
(86) International application number: PCT/IB2007/052701
(87) International publication number: WO 2008/012709

(56) References cited:
- WO-A-2006/137955
- WO-A-2007/070118
- DE-A1- 10 219 296
- US-A1- 2006 116 442
- US-A1- 2007 161 510
- US-B1- 6 290 977
- MACLAREN D C ET AL: "Dye-developer interactions in the crystal violet lactone-lauryl gallate binary system: implications for thermochromism" JOURNAL OF MATERIALS CHEMISTRY, THE ROYAL SOCIETY OF CHEMISTRY, CAMBRIDGE, GB, vol. 13, no. 7, 2003, pages 1695-1700, XP009096605 ISSN: 0959-9428

## Description

### BACKGROUND OF THE INVENTION

Various compositions are used to prepare the skin prior to surgery. Skin preparations or "preps" are used to remove some level of microbial load on the skin prior to making an incision. Skin sealant materials are used to protect patients from bacterial infections associated with surgical site incisions and insertion of intravenous needles. Skin preps are applied to the skin and allowed to dry to maximize effectiveness for reducing microorganisms. After the skin prep has dried, the sealant may be applied directly to the skin in liquid form. The sealant forms a coherent film with strong adhesion to the skin through various techniques based on the chemistry of the sealant composition.

Skin preps currently are predominantly povidone-iodine or chlorhexidine gluconate based formulations and may contain alcohol for fast drying and more effective killing of organisms. Time constraints in the operating room and the lack of an indicator that the prep has dried often result in the skin remaining wet when draping and/or surgery begin, creating the possibility of infection.

Skin sealants now use a polymer composition that dries to form a film through evaporation of a solvent, for example. Other skin sealants contain monomeric units that polymerize in situ to from a polymeric film. Cyanoacrylate sealants containing 2-cyanoacrylate monomer are an example of the latter type wherein the monomer polymerizes in the presence of a polar species such as water or protein molecules to form an acrylic film. The resulting film formed serves to immobilize bacterial flora found on the skin and prevents their migration into an incision made during a surgical procedure or skin puncture associated with insertion of an intravenous needle.

Skin sealants may contain additives such as plasticizing agents to improve film flexibility and conformance, viscosity modifiers to aid in application of the liquid composition, free radical and anionic scavengers to stabilize the product prior to use, biocidal agents to kill immobilized bacteria under the film, and the like.

Skin sealants have also been formulated with colorants to help the user apply the liquid composition uniformly to the skin, especially when large areas are to be covered. There are several problems, however, with existing colorants; addition of a colorant directly to the liquid skin sealant composition can negatively impact both in situ polymerization rates and the conversion reaction, in the case of cyanoacrylate compositions, or evaporation rates and the coalescence process in the case of polymer solution compositions. In addition, known colorants do not provide a visual cue to indicate curing of the composition has been completed. Lastly, after completion of the surgical procedure, the colorant in the sealant can obscure the wound site, making it difficult to detect redness associated with surgical site infections, brusing or leakage.

It is clear that there exists a need for a colorant that is compatible with skin preps and skin sealants, that provides a visual cue to indicate coverage area and/or curing and that does not obscure the wound site.

### SUMMARY OF THE INVENTION

In response to the foregoing difficulties encountered by those of skill in the art, we have discovered that thermochromic dyes may be used to indicate that a skin sealant has been applied. These dyes provide a color change in response to temperature changes, whether associated with curing or simple skin contact, that is visible to the unaided eye and does not negatively affect the skin sealant film formation. In addition, the dyes do not obscure the wound site after surgery since the color change is reversible and can be triggered by application of heating and cooling to create a clear film.

Generally, such thermochromic systems include at least two chemical components - a leuco dye and a color developer. The leuco dyes, which are weak organic bases, change from colored to colorless (clear) upon heating and normally function over a 5 to 15 °C temperature range. Changing temperature shifts the equilibrium between the colored or protonated form of the dye, where the proton is generally donated by the color developer (*e.g*. a weak acid) and the unprotonated or colorless form. Examples of leuco dyes include spirolactones such as fluorans or crystal violet lactone, spiropyrans, fulgides, and the like.

While the leuco dye itself will respond to temperature shifts with a color change, encapsulating the leuco dye with a color developer and optionally a polar solvent within a particle serves to isolate the components from the surrounding environment and produces a reversible thermochromic system. So, for example, a leuco dye could be combined with a color developer (i.e., a material that will facilitate protonation of the leuco dye) in a suitable solvent to produce the color changing system and then be encapsulated within, for example, a melamine formaldehyde shell.

### DETAILED DESCRIPTION OF THE INVENTION

The present invention involves the use of thermochromic, i.e., temperature responsive, dyes in a composition to be applied to the skin. Generally speaking, the composition is applied to mammalian skin. The dyes are colored when applied at a temperature below their transition temperature and become colorless above their transition temperature. The reaction is reversible.

Thermochromic dyes, more particularly, leuco dyes, are made from a colorant and an organic acid component in a phase change solvent. These dyes can be encapsulated within a polymeric microcapsule for ease of use.

Leuco dyes can have a reversible change in color with temperature such that the composition is colored when cool and clear or colorless when heated above the transition temperature of the solvent. The leuco dyes, which are weak organic bases, change from colored to colorless (clear) upon heating and normally function over a 5 to 15 °C temperature range. Changing temperature shifts the equilibrium between the colored or protonated form of the dye, where the proton is generally donated by the color developer (*e.g*. a weak acid) and the unprotonated or colorless form. Examples of leuco dyes include spirolactones such as fluorans or crystal violet lactone, spiropyrans, fulgides, and the like.

Leuco dyes generally complete a color change within a 3 to 4 °C temperature range. If the transition temperature of the leuco dye is reported as 30 °C, for example, the color change would occur between approximately 28 and 32 °C. The wide transition temperature range of Leuco dyes makes them generally unsuitable for the close monitoring of skin temperature, unlike liquid crystal technology, for example, since leuco dyes do not provide a unique temperature corresponding to a specific temperature of the skin surface. Thus the utility of leuco dyes for monitoring skin health and locating "hot spots" indicating possible infection on the skin is virtually nonexistent. The invention herein is therefore essentially free of liquid crystals.

Leuco dyes are available in a number of colors such as red, blue, green, yellow, and black and can be tailored by judicious selection of the polymeric precursor chemistries and emulsion polymerization conditions to produce the desired color change.

Microencapsulation serves to protect the components of the leuco dye from their surroundings, to ensure proper functioning and to prevent diffusion of the phase change solvent when it is present in liquid form. Microencapsulated leuco dyes are available from Color Change Corporation of Streamwood, Illinois, US, Chromatic Technologies, Inc. of Colorado Springs, Colorado, US, Matsui International Company Inc., Gardena, CA, US and Thermographic Measurement Co. of the United Kingdom. Adjustment of the thickness and thermal conductivity of the microcapsule shell materials results in changing the responsiveness of the leuco dye to the temperature change. The microcapsules are generally 3 to 5 microns in diameter though they can be made smaller than 1 micron. In the case of skin sealants, the proper selection of functional groups on the outer surface of the microcapsule can allow the capsules to be reacted into the skin sealant film during the curing reaction in order to prevent migration of the capsules into the wound site.

The skin sealant to which the leuco dye is added will be used on human or animal skin. Accordingly, the transition temperature must be between standard room temperature (20 - 24 °C) and that of the skin (30 - 35 °C). A transition temperature between these two ranges functions well, e.g. between about 24 and 35 °C or more particularly between about 26 and 33 °C or more particularly 30 and 31 °C.

When the sealant dye composition is spread on the skin it is applied at a temperature below the transition temperature of the dye and is initially colored so that the person applying the composition can see where it is being applied. As the composition cures, the temperature of the composition will rise due to the heat generated from the curing reaction as well as from conduction from the skin onto which it was applied. If the curing method is by evaporation of solvent then the film will be cooled through evaporative cooling of the solvent. Once the solvent is gone the film will be warmed by the skin causing the color change to occur. After a period of time the sealant temperature will be sufficiently raised such that the sealant becomes substantially clear or colorless. (For purposes of discussion herein, the transition between a colored form of the composition and a substantially "clear" or colorless form of the composition is considered a color change.) This will allow excellent visibility of the incision or wound site through the sealant. Should the skin temperature drop below the transition temperature, the composition will again become colored since the reaction is reversible.

As noted above, color developers are weak acids (i.e., proton donors or electron acceptors). Examples of such components include bisphenol A, octyl *p-*hydroxybenzoate, methyl *p*-hydroxybenzoate, 1,2,3-triazoles, 4-hydroxycoumarin derivatives, and the like.

A third component for an organic-dye system (such as a leuco dye) may generally be a polar solvent such as an alcohol, ester, ketone, or ether. Examples include lauryl alcohol (i.e., 1-dodecanol), cetyl alcohol (i.e., 1-hexadecanol), methyl stearate and butyl stearate. The solvent provides additional control over the reversibility of the system and may be a fatty alcohol or lipid. A lipid like methyl stearate, for example, which is opaque when a solid but transparent when a liquid, dissolves the leuco dye and color developer at higher temperature, thereby disfavoring protonation of the leuco dye (i.e., favoring the colorless state). At lower temperatures, methyl stearate solidifies and the leuco dye and the color developer are frozen in place, thereby favoring protonation of the dye and formation of the colored state.

A second, color one to color two visual change can be obtained by this invention by mixing a thermochromic dye with a permanent (non-temperature sensitive) color dye to obtain a different starting color. Mixing a blue thermochromic dye with a permanent yellow color dye for example, produces a green color that can be used as the starting or initial color. On application of this green colored skin composition to the skin, the composition is warmed by the skin and the blue thermochromic dye component turns colorless, leaving yellow to be the resultant color. (This composition could be formulated such that the yellow disappeared and left blue as well.) This informs the user that the composition has cured and yet the color is pale enough for the caregiver to fully assess the wound. Virtually any combination of primary colors is possible including purple to red or blue, brown to green or red, turquoise to hot pink and black and virtually any color like, for example, yellow.

An effective amount of microencapsulated dye is physically blended with a composition to be applied to skin to produce the thermochromic skin sealant composition. The amount needed is that to produce a blend having between 0.01 and 30 weight percent of the microencapsulated dye, more particularly between 1 and 20 weight percent dye, still more particularly between 2 and 10 weight percent.

### EXAMPLES

### Example 1: Commercial 2-cyanoacrylate glue

Standard Loctite® super glue adhesive (cyanoacrylate based from Henkel Adhesives, Inc., Avon OH) was used as the model adhesive. The thermochromic powders (microencapsulated colorants obtained from Color Change Corp., Streamwood, IL) were added to the adhesive and mixed by hand for 2 minutes. The thermochromic powders had temperature transitions of 29 °C and 31 °C. A series of liquids were prepared having differing concentrations of the thermochromic dyes in the range of:
4-6% wt/wt for black 29 °C dye
5-8% wt/wt for blue 31 °C dye
5-8% wt/wt for red 31 °C dye
The mixtures were then spread onto transparency films by smearing with a flat knife to give a coating with visible color intensity to still be visible when placed over skin. The adhesive was allowed to dry and then the researchers hand was placed onto the adhesive area for one minute. The color of the adhesive was discharged resulting in the adhesive patch to become colorless and transparent.

### Example 2: 2-cyanoacrylate skin sealant formulations

In the following examples InteguSeal® Clear skin sealant (MedLogic Global Limited, Plymouth, Devon, England, UK and Aspire Biotech, Inc. Colorado Springs, CO) was used. It comprises 80% wt/wt n-butyl cyanoacrylate and 20% wt/wt tributyl o-acetylcitrate. Samples of this adhesive were used and the colorant added directly to the formulation with mixing.

### 1.) Red to Colorless

To 5 ml of the InteguSeal® Clear skin sealant was added 0.1g (2% wt/wt) of red encapsulated thermochromic powder (31 °C, Leuco Dye - powder [LD-P]) from Color Change Corporation, Streamwood IL. The mixture was stirred for 1 minute and then applied to the forearm skin of a volunteer using a cosmetic sponge to provide a thin uniform coating. The skin sealant was observed to be red upon application providing the visual queue to the area being covered. After one minute the sealant had cured and the color was discharged, leaving a colorless area.

When an ice bag was applied to the skin sealant area the red color reappeared.

### 2.) Blue to Colorless

To 5 ml of the InteguSeal® Clear skin sealant was added 0.15g (3% wt/wt) of the blue encapsulated thermochromic powder (33 °C, Leuco Dye - powder, LD-P) from Color Change Corporation, Streamwood IL. The mixture was stirred for 1 minute and then applied to a volunteer's forearm using a cosmetic sponge. The skin sealant was clearly seen as a blue area which turned colorless after 3 minutes. The sealant was tested by rubbing and found to be cured and dry.

When the area was cooled by an ice bag the blue color returned after 20 seconds of applying the ice to the surface.

### 3.) Black to Colorless

To 5 ml of the InteguSeal® Clear skin sealant was added 0.1 g (2% wt/wt) of the black encapsulated thermochromic powder (33 °C, Leuco Dye - powder, LD-P) from Color Change Corporation, Streamwood IL. The mixture was stirred for 1 minute and then applied to a volunteer's forearm using a cosmetic sponge. The skin sealant was clearly seen as a black area which turned colorless after 1 minute. The sealant was tested by rubbing and found to be cured and dry. When the area was cooled by an ice bag the black color returned after 20 seconds of applying the ice to the surface.

### 4.) Purple to Colorless

To 5 ml of the InteguSeal® was added 0.1g (2% wt/wt) of the red encapsulated thermochromic powder (31 °C, Leuco Dye - powder, LD-P) and 0.1g (2% wt/wt) of the blue encapsulated thermochromic powder, both from Color Change Corporation, Streamwood IL. The mixture was stirred for 1 minute and then applied to a volunteer's forearm using a cosmetic sponge. The skin sealant was clearly seen as a purple area which turned colorless after 3 minutes. The sealant was tested by rubbing and found to be cured and dry. When the area was cooled by an ice bag the purple color returned after 20 seconds of applying the ice to the surface.

### 5.) Color Changing Temporary Pattern, e.g. Tattoo or logo (not claimed)

To illustrate the potential of a hidden temporary pattern like a tattoo, logo or transfer the following example was conducted. A stencil pattern in the shape of the Kimberly-Clark Corporation logo was printed onto a clear plastic sheet and then carefully cut out. This was placed onto the forearm of a volunteer and the black colored skin adhesive from example 3 was applied to the open area of the stencil. A black colored image of the logo was left on the skin. After 2 minutes the sealant was dry and the image color discharged to leave an invisible area. When an ice bag was applied to the area the logo image appeared. It should be understood that this would also appear if the arm were placed into water colder than the skin surface, such as a swimming pool or cold environment. This may find utility as a possible indicator of frostbite. This may also be used as an enjoyable diversion or playtime activity for children.

### Example 3: Skin prep (not claimed)

### Blue to Colorless

To 10 ml of Chloraprep® One-Step skin preparation (2 wt%/v chlorhexidine gluconate in isopropanol 70 %v/v) was added 0.1g of the blue encapsulated thermochromic powder (31 °C, Leuco Dye - powder, LD-P) from Color Change Corporation, Streamwood IL. The mixture was stirred for 10 minutes and then applied to a volunteer's hand and rubbed to disperse the liquid over a 5 cm diameter area. The skin prep was clearly seen as a blue area which turned colorless very quickly as the alcohol evaporated.

## Claims

1. A composition for application to the skin comprising a thermochromic leuco dye, wherein said composition is a skin sealant comprising cyanoacrylate.

2. The composition of claim 1 wherein said dye is microencapsulated.

3. The composition of claim 2 wherein said dye is present in an amount of between about 0.01 and 30 weight percent on a dry basis.

4. The composition of claim 1 that changes color in less than 4 minutes after application to skin.

5. The composition of claim 1 that changes color in less than 2 minutes after application to skin.

6. The composition of claim 1 that changes color in less than 1 minute after application to skin.

7. The composition of claim 1 further comprising a non-temperature sensitive dye.

8. A method of indicating the surface coverage of a composition to be applied to the skin and of indicating that said composition has cured, comprising the steps of applying to an area of skin said composition having a first color and comprising a thermochromic leuco dye, and observing said color until said color changes to a second color, wherein the area of skin covered by said first color indicates the surface coverage and said second color indicates the composition has cured, wherein said composition is a skin sealant comprising cyanoacrylate.

9. The method of claim 8 wherein said second color is clear.

10. The method of claim 8 wherein said composition further comprises a non-temperature sensitive dye.

## Patentansprüche

1. Zusammensetzung zur Anwendung auf der Haut, umfassend einen thermochromen Leuco-Farbstoff, wobei die Zusammensetzung ein Hautversiegelungsmittel ist, das Cyanoacrylat umfasst.

2. Zusammensetzung nach Anspruch 1, wobei der Farbstoff mikroverkapselt ist.

3. Zusammensetzung nach Anspruch 2, wobei der Farbstoff in einer Menge zwischen etwa 0,01 und 30 Gewichtsprozent auf trockener Basis vorhanden ist.

4. Zusammensetzung nach Anspruch 1, die die Farbe in weniger als 4 Minuten nach der Anwendung auf der Haut wechselt.

5. Zusammensetzung nach Anspruch 1, die die Farbe in weniger als 2 Minuten nach der Anwendung auf der Haut wechselt.

6. Zusammensetzung nach Anspruch 1, die die Farbe in weniger als 1 Minute nach der Anwendung auf der Haut wechselt.

7. Zusammensetzung nach Anspruch 1, weiterhin unfassend einen temperaturunempfindlichen Farbstoff.

8. Verfahren zum Anzeigen der Oberflächenbedeckung einer Zusammensetzung, die auf der Haut angewendet werden soll, und zum Anzeigen, dass die Zusammensetzung geheilt hat, umfassend die Schritte der Anwendung der Zusammensetzung, die eine erste Farbe aufweist und einen thermochromen Leuco-Farbstoff umfasst, auf einem Hautbereich und Beobachten der Farbe, bis sich die Farbe zu einer zweiten Farbe ändert, wobei der Hautbereich, der von der ersten Farbe bedeckt ist, die Bedeckung der Oberfläche anzeigt, und die zweite Farbe anzeigt, dass die Zusammensetzung geheilt hat, wobei die Zusammensetzung ein Hautversiegelungsmittel ist, das Cyanoacrylat umfasst.

9. Verfahren nach Anspruch 8, wobei die zweite Farbe farblos ist.

10. Verfahren nach Anspruch 8, wobei die Zusammensetzung weiterhin einen temperaturunempfindlichen Farbstoff umfasst.

## Revendications

1. Composition à appliquer sur la peau, comprenant un leuco-colorant thermochromique, ladite composition étant un agent de protection cutanée comprenant un cyanoacrylate.

2. Composition selon la revendication 1, dans laquelle ledit colorant est microencapsulé.

3. Composition selon la revendication 2, dans laquelle ledit colorant est présent en une quantité comprisse entre environ 0,01 et 30 pour cent en poids sur une base sèche.

4. Composition selon la revendication 1, qui change de couleur en moins de 4 minutes après application sur la peau.

5. Composition selon la revendication 1, qui change de couleur en moins de 2 minutes après application sur la peau.

6. Composition selon la revendication 1, qui change de couleur en moins de 1 minute après application sur la peau.

7. Composition selon la revendication 1, comprenant en outre un colorant non sensible à la température.

8. procédé pour indiquer la couverture de surface d'une composition à appliquer sur la peau et pour indiquer que ladite composition a durci, comprenant les étapes d'application, sur une zone de la peau, de ladite composition ayant une première couleur et comprenant un colorant leuco thermochromique, et d'observation de ladite couleur jusqu'à ce que ladite couleur se change en une seconde couleur, dans lequel la surface de peau recouverte par ladite première couleur indique la couverture de surface et ladite seconde couleur indique que la composition a durci, dans lequel ladite composition est un agent de protection cutanée comprenant un cyanoacrylate.

9. Procédé selon la revendication 8, dans lequel ladite seconde couleur est claire.

10. Procédé selon la revendication 8, dans lequel ladite composition comprend en outre un colorant non sensible à la température.
